# EUROPEAN PATENT SPECIFICATION

(11) **EP 0 957 920 B1**
(45) Date of publication and mention of the grant of the patent: **19.12.2001**
(21) Application number: 97941110.5
(22) Date of filing: 23.09.1997
(51) Int. Cl.: A61K 31/555

(54) **REDUCING TOXIC EFFECTS OF CARBOPLATIN USING DITHIOETHERS**
MINDERUNG DER TOXISCHEN EFFEKTE DES CARBOPLATINS DURCH VERWENDUNG VON DITHIOETHERN
REDUCTION DES EFFETS TOXIQUES DU CARBOPLATINE PAR UTILISATION DE DITHIOETHERS

(30) Priority: 23.09.1996 US 26430 P
(43) Date of publication of application: 24.11.1999
(73) Proprietor: BIONUMERIK PHARMACEUTICALS, INC., San Antonio, Texas 78229 (US)
(72) Inventor: HAUSHEER, Frederick, Herman, Fair Oaks Ranch, TX 78015 (US); HARIDAS, Kochat, San Antonio, TX 78248 (US); REDDY, Dasharatha, Gauravaram, San Antonio, TX 78248 (US); SEETHARAMULU, Peddaiahgari, San Antonio, TX 78248 (US); MURALI, Dhanabalan, San Antonio, TX 78230 (US)
(74) Representative: Lucas, Brian Ronald
(86) International application number: GB9702582
(87) International publication number: WO9811898

(56) References cited:
- HAUSHEER F ET AL: "BNP7787: A novel antitumor potentiating drug which protects against cisplatin and carboplatin toxicities" PROCEEDINGS OF THE AMERICAN ASSOCIATION FOR CANCER RESEARCH ANNUAL MEETING, 38 (0). MARCH 1997. 311-312., XP002052163
- PETERS G.J. ET AL: "Protection of normal tissues from the cytotoxic effects of chemotherapy and radiation by amifostine (WR-2721): Preclinical aspects" EUROPEAN JOURNAL OF CANCER PART A: GENERAL TOPICS, 1995, 31/SUPPL. 1 (S1-S7), UNITED KINGDOM, XP002052164
- TRESKES M. ET AL: "The chemical reactivity of the modulating agent WR2721 (ethiofos) and its main metabolites with the antitumor agents cisplatin and carboplatin" BIOCHEM. PHARMACOL., 1991, 42/11 (2125-2130), UNITED KINGDOM, XP002052165
- VAN DER VIJGH W.J.F. ET AL: "Protection of normal tissues from the cytotoxic effects of chemotherapy and radiation by amifostine (Ethyol): Preclinical aspects" SEMIN. ONCOL., 1994, 21/5 SUPPL. 11 (2-7), USA, XP002058835

## Description

### FIELD OF THE INVENTION

This invention relates to reducing the toxicity of carboplatin (cis-diammine-1,1-cyclobutanedicarboxylato-platinum II, CBDCA, JM-8 and NSC 241240), using a dithioether as a protective agent.

### BACKGROUND OF THE INVENTION

Carboplatin (hereinafter also referred to as "CBDCA" or CBP") is a widely used anticancer drug which is normally used in combination with other anticancer drugs in the treatment of cancers of the lung, head and neck, ovary, esophagus, bladder, testis, and others. One of the most important and common dose limiting toxicities of carboplatin is hematological toxicity. In particular, myelosuppression (depression of blood elements formed within bone marrow) is often manifested in the form of thrombocytopenia, neutropenia, leukopenia, and various forms of anemia. The other major carboplatin-induced toxicity is gastro-intestinal (causing nausea and vomiting).

In order for carboplatin to react with certain nucleic acid sequences in cellular DNA, it must first undergo chemical conversion to an active species by the partial or complete displacement of the cyclobutanedicarboxylato (CBDC) ligands, respectively, by chloride. The chloro and dichloro species are believed to act against cancer cells by reacting with the imidazole nitrogens on DNA. These chloro species are believed to be metabolised in vivo to active hydroxy species.

Carboplatin, unlike cisplatin, is relatively stable in the body. Its cyclobutanedicarboxylato (CBDC) group makes it much less susceptible to displacement by incoming nucleophiles. It is less active than cisplatin towards DNA. Indeed, it is generally given in combination with other anti-cancer drugs. Although less prone to cause the nephrotoxicity associated with cisplatin, it is highly myelotoxic and has gastrointestinal toxicity. It might at first be thought that carboplatin, with its active chloro species, should behave similarly to cisplatin and therefore be further metabolised in the same way. However, this is not so: the body cells most adversely affected by carboplatin-induced toxicity (bone marrow and GI tract cells) are different from those most adversely affected by cisplatin (kidney cells). Thus, the metabolic species responsible for the toxicity cannot be the same. Finding a protective agent for carboplatin therefore represents a new and separate problem from that presented by cisplatin.

Treskes *et al*., Biochem. Pharmacol. 42, 2125-2130 (1991) are concerned mainly with the mechanism by which the compound WR2721, ethiofos, of formula H₂N-(CH₂)₃-NH-(CN₂)₂-S-PO₃H₂, protects against the side effects of cisplatin and carboplatin. The paper notes that DDTC, diethyldithiocarbamate "may" lower myelotoxicity, but that clinical studies were disappointing due to toxic side effects of DDTC itself and insufficient protection against carboplatin-induced myelotoxicity.

### SUMMARY OF THE INVENTION

It has now been found that a dithioether having the formula R₁-(CH₂)ₙ-S-S-(CH₂)ₘ-R₂ (I) wherein:
each of R₁ and R₂ individually is SO₃H or PO₃H₂; and
each of m and n is individually 1, 2, 3 or 4; or a pharmaceutically acceptable salt thereof, is a suitable protective agent for carboplatin.

This invention can be represented in different ways according to local patent law. Thus, it includes the use of the dithioether in the manufacture of a medicament for administration in combination with carboplatin to a patient, at substantially the same time or sequentially, whereby the dithioether and the carboplatin become co-present in the blood of the patient and the dithioether serves to reduce the toxicity of the carboplatin. Also within the invention is a combination suitable for sequential administration to patients with cancer, comprising carboplatin and the dithioether.

The preferred dithioether is sodium 2,2'-dithiobis(ethanesulfonate), herein abbreviated to dimesna.

The invention is useful in relation to any cancer treatable by a therapy consisting of or including administration of carboplatin, especially the cancers specifically listed above.

The invention also includes a composition suitable for administration to patients with cancer, comprising carboplatin and a dithioether as defined above, especially in the form of a sterile injectable solution, preferably of pH 2 to 6.

### DESCRIPTION OF THE PREFERRED EMBODIMENTS

The carboplatin formulation used in the invention will typically be a solution. It can take any form appropriate to or conventional for formulation of carboplatin. The type of formulation will of course depend on the route of administration, which will normally be parenteral, especially intravenous, and preferably by injection. The preferred solvent is aqueous, since carboplatin has a water-solubility of 14 mg/mL. The formulation will normally contain from 0.05 mg/mL up to the maximum solubility of carboplatin. It may also include mannitol (a preservative) . It may contain other excipient(s) and/or diluent(s).

The carboplatin formulation will normally have a pH of from 2 to 6; a neutral pH of about 7 is much less preferred. Any pharmaceutically acceptable acid, including hydrochloric acid, may be used to adjust the pH. However, a formulation which is substantially free of added chloride ions, or, at least, free of chloride ions from added sodium chloride, has been shown to have improved stability. Cisplatin, by contrast, is preferably formulated in a chloride ion solution, such as isotonic or hypertonic saline for injection.

The dithioether protective agent is formulated separately from the carboplatin. For oral administration, it may be formulated as a tablet, capsule, caplet, colloidal suspension or solution, or other form which is easily ingested by the patient. For parenteral administration it is preferably formulated as a sterile injectable solution, as described above. The oral and/or parenteral formulation may be stored as an aqueous solution or as a lyophilized powder suitable for reconstitution with water or another solvent.

Hereinafter, the dithioether will be discussed mainly with reference to disodium 2,2'-dithiobis(ethanesulfonate) (dimesna), but the person skilled in the art will readily be able to use the principles of this invention in relation to other dithioethers of formula (I), especially a compound of formula (I) in which m and n are from 1 to 3, in the form of a sodium salt.

The preferred dithioethers are the sulfonates, especially the disodium salts thereof, but including the monosodium, monopotassium, sodium-potassium, dipotassium, calcium and magnesium salts of the sulfonate. Further, since it is the dithio group which must provide a reactive nucleophile for quenching the reactive species of carboplatin and the sulfonate group confers water-solubility on the molecule, it follows that the sulfonate group could be replaced by phosphonate. Thus, disodium and tetrasodium 2,2'-dithiobis(ethanephosphonates) are also preferred dithioethers for use in the invention.

For the dithioether formulation, the preferred solvent is aqueous, since the dithioethers are also water-soluble, e.g. up to 300 mg/mL for dimesna. The concentration of dithioether is normally from 1 mg/mL up to the maximum solubility. In principle, higher amounts of the dithioether are usable, e.g. up to 500 mg/mL, although, of course, solubility problems may arise. The formulation may contain excipient(s) and/or diluent(s).

The carboplatin/dithioether combination can be administered to human or non-human patients as a treatment for various types of cancer, as described in the effectiveness profile for carboplatin. It may be administered as a "single drug therapy" (carboplatin being the sole cytotoxic or anti-cancer therapeutic agent) or in combination with other cytotoxic, anti-cancer or other chemotherapeutic agents.

Typically, the carboplatin and the dithioether formulations will be prepared as for intravenous injection in sterile, single-dose containers. Both could be administered orally.

Preferably the dithioether is administered before the carboplatin, especially from 5 minutes to 1 hour before. For dimesna, from 15 to 30 minutes before has been found very effective. Administration of the combination of carboplatin and the dithioether may require adjustments in one or both of timing and/or dosage. The goal in the treatment is to match the peak in vivo concentration of the dithioether with that of the toxic metabolites of carboplatin. Although carboplatin reacts slowly in vivo to produce the active nucleophilic species which ultimately cause damage in certain cells, especially bone marrow and GI tract cells, it has been found that at least the greater proportion of the dose of dithioether should be given before the carboplatin, although it will sometimes be helpful to give the remaining, smaller proportion, of the dose of dithioether after the carboplatin, in order to combat the effects of slowly-generated or long-lasting active nucleophilic species of carboplatin. Carboplatin has a relatively long half-life in the body. Desirability and necessity of additional doses of protective agent is determined by carefully monitoring the patient's excretion of platinum to estimate the rate of drug elimination from the body.

The invention includes the possibility of administering at least a part of the dithioether in the form of a composition comprising the carboplatin and the dithioether components, preferably as an aqueous solution of pH 2 to 6. Features of preference of such compositions are as recited above for the individual components.

The carboplatin can be administered in any conventional dose. It may be possible to exceed the conventional dose of carboplatin, as this is frequently limited by the toxicity problem which the present invention mitigates. The carboplatin dose will normally be in the range 0.3 to 45 mg/kg (a corresponding dose of dithioether would then be from 20 to 2500 mg/kg, increasing roughly proportionately with the carboplatin dose). In terms of body surface area, ranges of 100 to 1000 mg/m² of carboplatin and 1000 to 40,000 mg/m² of dithioether are suggested.

Since the toxicity of the protective agent is very low (the parenteral and oral LD₅₀ values for all of the dithioethers of formula I are generally higher than that of common table salt, and all are rapidly eliminated through excretion in the urine), large amounts of the protective agent may be given either orally or parenterally to provide constant and safe protection against any residual carboplatin toxicity. Thus, typically the weight ratio of carboplatin to dithioether used in the therapy is from 6:1 to 1000:1, most especially 25:1 to 700:1.

The following non-limiting Examples illustrate the invention. The vials referred to are "amber vials", which protect the carboplatin from exposure to light. Although Examples 1 to 4 relate to solutions of carboplatin and the dithioether together, it will be appreciated that they can be formulated separately, with the carboplatin at acidic pH and each active component at the concentration indicated in the solutions of the Examples.

### EXAMPLE 1

### (a) Preparation of 2,2' -dithiobis (ethanesulfonate) (dimesna)

Disodium 2,2'-dithiobis (ethanesulfonate) was prepared by oxidizing 2-mercaptoethanesulfonate in water with an equimolar amount of iodine as previously reported by L. Lamaire and M. Reiger, J. Org. Chem. 26, 1330-1, (1961). The other sulfonate and phosphonate dithioethers of formula (I) can be prepared analogously.

### (b) Stability of dimesna

50 mg of the dimesna thus prepared were dissolved in 1 mL of water and the pH of the solution adjusted to 1.5, 2.0, 3.0, 4.0, 5.0 and 6.0 by adding 1N hydrochloric acid in water or the pH adjusted to 8.0 and 9.0 by adding 1 N sodium hydroxide in water. The solution was then stirred for 24 hours at room temperature, the water was removed at reduced pressure and the residue dissolved in spectral grade D₂O. The proton MMR spectrum gave only peaks corresponding to the starting material. Heating the pH 1.5 solution to 100°C for 10 minutes gave no change in the proton NMR spectrum. These data indicate that dimesna is stable in aqueous solution at pH 1.5 to 9.0.

### (c) Preparation of a sterile solution of carboplatin and dimesna

Pure hydrochloric acid (99.999%) was added to a sterile, injectable, Lactated Ringer's (LR) solution (US Pharmacopoeia grade), to give a pH in the range 2.0 to 6.0. 1 mg of pure carboplatin/mL of the above LR solution was added and allowed to completely dissolve by agitation (1500-2500 rpm) at room temperature, for approximately 60 to 90 minutes in the dark. Then, 15 mg of dimesna, prepared above, per mL of solution were added and the mixture agitated until complete dissolution occurred. The final pH was adjusted to within the range pH 2.0 to 6.0 by adding further pure hydrochloric acid. The solution was sterilized by filtration through a sterile 0.2 micrometre filter (obtained from VWR Scientific) and stored in sterile injection vials. Each vial contained approximately 0.9 mg of carboplatin and 14.3 mg of dimesna per mL of solution.

### EXAMPLE 2

To a sterile injectable aqueous solution of LR solution(USP grade) were added 15 mg of dimesna/mL of solution. The dimesna was allowed to dissolve completely by agitation (1500-2500 rpm) at room temperature, for 5-10 minutes. The pH of the solution was adjusted to within the range 2.0 to 6.0 by adding pure (99.999%) hydrochloric acid. 1 mg/mL of dimesna solution of pure (98.0%) carboplatin was added and the mixture agitated in the dark until complete dissolution occurred. The remaining steps were as in Example 1 (c), giving a solution of the same approximate composition.
Each vial contained approximately 1.0 mg of carboplatin and 14.3 mg of dimesna per mL of injection solution.

### EXAMPLE 3

Example 1(c) was repeated except that 0.5 mg/mL of carboplatin and 30 mg/mL of dimesna were used. Each vial contained 0.5 mg of carboplatin and 12.9 mg of dimesna per mL of injection solution.

### EXAMPLE 4

Example 1(c) was repeated except that pure mannitol (99+ % purity, from Aldrich Chemical Company) was dissolved in the LR solution, to give a concentration of 1.0% w/v mannitol, and also that 30 mg/mL of dimesna were used. Each vial contained approximately 1.0 mg of carboplatin and 12.9 mg of dimesna per mL of injection solution.

### EXAMPLE 5

### Use of Dimesna to Reduce Carboplatin Toxicity

Experiments were performed to determine the efficacy of dimesna in reducing the toxicity of carboplatin in adult beagle dogs. Toxic effects were tested at varying dose levels of carboplatin, with or without administration of dimesna. The carboplatin used was an aqueous solution from vials of "Paraplatin" (Bristol Myers Squibb) and injected i.v.
by a slow drip over 5 to 10 minutes. Lyophilised dimesna was reconstituted in water and injected i.v. 30 minutes before the carboplatin.

Table 1 gives the dosing schedule followed in the experiments, with two animals, one male and one female in each group.

**Table 1**

| Group | Dimesna (mg/kg) | Carboplatin (mg/kg) |
|---|---|---|
| 1 | 0 | 45 |
| 2 | 2000 | 45 |
| 3 | 0 | 30 |
| 4 | 2000 | 30 |
| 5 | 0 | 20 |
| 6 | 2000 | 20 |
| 7 | 0 | 13 |
| 8 | 2000 | 13 |
| 9 | 2000 | 0 |

The animals were closely observed after the dose was administered, and euthanized on day 30 for tissue necropsy. Tissues were trimmed, processed, and stained slides were prepared of the following: thymus, heart, lung, stomach, duodenum, jejunum, colon, pancreas, liver, kidney, urinary bladder, testis, ovary, spleen, mesenteric and mandibular lymph nodes, bone marrow, ischiatic nerve, and all gross lesions. Lesions found were graded from one to five based upon severity.

### Results

Three dogs, the two from control Group 1 which received only a 45 mg/kg dose of carboplatin, and the female from control Group 3 (30 mg/kg carboplatin only), died or were sacrificed because of their moribund condition prior to day 30. Most of the other dogs from control Groups 3, 5 and 7 exhibited moderate to severe cellular depletion of femoral bone marrow, moderate to severe lymphoid depletion of the thymus, and other microscopic lesions, particularly in the gastrointestinal tract. The dogs from the Groups 2, 4, 6 and 8, which were given dismesna, all survived for the duration of the experiment. Dogs from Groups 4, 6 and 8 showed no cellular depletion of the femoral bone marrow or lymphoid depletion in the thymus. The dogs from Group 2, which received in essence, a lethal dose of carboplatin, both survived and exhibited only mild lymphoid depletion, with no evident cellular depletion of bone marrow. Group 9 control dogs which received only dimesna showed no physiological changes.

## Claims

1. Use of a dithioether having the formula
R₁-(CH₂)ₙ-S-S-(CH₂)ₘ-R₂ (I)
wherein:
each of R₁ and R₂ individually is SO₃H or PO₃H₂; and
each of m and n is individually 1, 2, 3 or 4;
or a pharmaceutically acceptable salt thereof, in the manufacture of a medicament for administration in combination with carboplatin to a patient, at substantially the same time or sequentially, whereby the dithioether and the carboplatin become co-present in the blood of the patient and the dithioether serves to reduce the toxicity of the carboplatin.

2. Use according to Claim 1, wherein the dithioether is to be administered to the patient prior to the administration of carboplatin.

3. Use according to Claim 2, wherein the dithioether is to be administered at a time from 5 minutes prior to 1 hour prior to the administration of carboplatin.

4. Use according to Claim 1, 2 or 3, wherein the dithioether and the carboplatin are to be administered either both intravenously or both orally.

5. Use according to Claim 1, 2, 3 or 4, wherein the weight ratio of dithioether to carboplatin to be administered is from 25:1 to 700:1.

6. Use according to Claim 1, 2, 3, 4 or 5, wherein the dithioether is a compound of formula (I) in which m and n are from 1 to 3, in the form of a sodium salt.

7. Use according to Claim 6, wherein the dithioether is dimesna, being the compound of formula (I) in which m and n are 2 and R₁ and R₂ are SO₃H, in the form of the disodium salt.

8. Use according to Claim 7, wherein the dimesna is to be administered from 15 to 30 minutes before the carboplatin.

9. A composition suitable for administration to patients with cancer, comprising carboplatin and a dithioether as defined in Claim 1, 6 or 7.

10. A composition according to Claim 9, in the form of a sterile injectable aqueous solution or suspension of pH 2 to 6.

11. A composition according to Claim 10, wherein the weight ratio of dithioether to carboplatin is from 25:1 to 700:1.

12. A composition according to Claim 9, 10 or 11, for use in cancer therapy.

13. A combination suitable for sequential administration to patients with cancer, comprising carboplatin and a dithioether as defined in Claim 1, 6 or 7.

14. A combination according to Claim 13, for use in cancer therapy.

## Patentansprüche

1. Verwendung eines Dithioethers mit der Formel
R₁-(CH₂)ₙ-S-S-(CH₂)ₘ-R₂
wobei:
R₁ und R₂ jeweils SO₂H oder PO₃H₂ sind und
m und n jeweils 1, 2, 3 oder 4 sind,
oder eines pharmazeutisch verträglichen Salzes davon bei der Herstellung eines Medikamentes für die im wesentlichen gleichzeitige oder aufeinanderfolgende Verabreichung in Kombination mit Carboplatin an einen Patienten, wodurch der Dithioether und das Carboplatin gleichzeitig im Blut des Patienten vorhanden sind und der Dithioether der Verminderung der Toxizität des Carboplatins dient.

2. Verwendung nach Anspruch 1, wobei der Dithioether dem Patienten vor der Verabreichung des Carboplatins verabreicht werden soll.

3. Verwendung nach Anspruch 2, wobei der Dithioether zu einem Zeitpunkt von 5 Minuten bis 1 Stunde vor der Verabreichung des Carboplatins verabreicht werden soll.

4. Verwendung nach Anspruch 1, 2 oder 3, wobei der Dithioether und das Carboplatin entweder beide intravenös oder beide oral verabreicht werden sollen.

5. Verwendung nach Anspruch 1, 2, 3 oder 4, wobei das zu verabreichende Gewichtsverhältnis von Dithioether zu Carboplatin von 25:1 bis 700:1 beträgt.

6. Verwendung nach Anspruch 1, 2, 3, 4 oder 5, wobei der Dithioether eine Verbindung der Formel (I), in der m und n 1 bis 3 sind, in Form eines Natriumsalzes ist.

7. Verwendung nach Anspruch 6, wobei der Dithioether Dimesna, das eine Verbindung der Formel (I) ist, in der m und n 2 sind und R₁ und R₂ SO₃H sind, in Form des Dinatriumsalzes ist.

8. Verwendung nach Anspruch 7, wobei das Dimesna 15 bis 30 Minuten vor dem Carboplatin verabreicht werden soll.

9. Zusammensetzung, die für die Verabreichung an Patienten mit Krebs geeignet ist, die Carboplatin und einen wie in den Ansprüchen 1, 6 oder 7 definierten Dithioether umfaßt.

10. Zusammensetzung nach Anspruch 9 in Form einer sterilen, injizierbaren, wäßrigen Lösung oder Suspension mit einem pH-Wert von 2 bis 6.

11. Zusammensetzung nach Anspruch 10, wobei das Gewichtsverhältnis von Dithioether zu Carboplatin von 25:1 bis 700:1 beträgt.

12. Zusammensetzung nach Anspruch 9, 10 oder 11 zur Verwendung bei der Krebstherapie.

13. Kombination, die für die aufeinanderfolgende Verabreichung an Patienten mit Krebs geeignet ist, die Carboplatin und einen wie in den Ansprüchen 1, 6 oder 7 definierten Dithioether umfaßt.

14. Kombination nach Anspruch 13 für die Verwendung bei der Krebstherapie.

## Revendications

1. Utilisation d'un dithioéther de formule
R₁-(CH₂)ₙ-S-S-(CH₂)ₘ-R₂ (I)
dans laquelle :
chacun de R₁ et R₂ représente individuellement SO₃H ou PO₃H₂ ; et
chacun de m et n représente individuellement 1, 2, 3 ou 4 ;
ou d'un sel pharmaceutiquement acceptable de celui-ci, dans la fabrication d'un médicament destiné à être administré conjointement avec du carboplatine à un patient, essentiellement simultanément ou séquentiellement, le dithioéther et le carboplatine étant alors présents simultanément dans le sang du patient et le dithioéther servant à réduire la toxicité du carboplatine.

2. Utilisation selon la revendication 1, dans laquelle le dithioéther doit être administré au patient avant administration du carboplatine.

3. Utilisation selon la revendication 2, dans laquelle le dithioéther doit être administré 5 minutes à 1 heure avant administration du carboplatine.

4. Utilisation selon la revendication 1, 2 ou 3, dans laquelle le dithioéther et le carboplatine doivent être administrés tous deux par voie intraveineuse ou orale.

5. Utilisation selon la revendication 1, 2,3 ou 4, dans laquelle le rapport en poids du dithioéther au carboplatine à administrer est de 25 :1 à 700 : 1.

6. Utilisation selon la revendication 1, 2, 3, 4 ou 5, dans laquelle le dithioéther est un composé de formule (I) dans laquelle m et n valent de 1 à 3, sous la forme d'un sel de sodium.

7. Utilisation selon la revendication 6, dans laquelle le dithioéther est le dimesna, un composé de formule (I) dans laquelle m et n valent 2 et R₁ et R₂ représentent SO₃H, sous la forme de son sel disodique.

8. Utilisation selon la revendication 7, dans laquelle le dimesna doit être administré 15 à 30 minutes avant l'administration du carboplatine.

9. Composition appropriée pour être administrée à des patients atteints d'un cancer, comprenant du carboplatine et un dithioéther tel que défini à la revendication 1, 6 ou 7.

10. Composition selon la revendication 9, qui se présente sous forme de solution ou suspension aqueuse stérile injectable ayant un pH de 2 à 6.

11. Composition selon la revendication 10, dans laquelle le rapport en poids du dithioéther au carboplatine est de 25 :1 à 700 : 1.

12. Composition selon la revendication 9, 10 ou 11, destinée à être utilisée dans le traitement thérapeutique du cancer.

13. Combinaison appropriée pour administration séquentielle à des patients atteints de cancer, comprenant du carboplatine et un dithioéther tel que défini dans la revendication 1, 6 ou 7.

14. Combinaison selon la revendication 13, destinée à être utilisée dans le traitement thérapeutique du cancer.
